# EUROPEAN PATENT APPLICATION

(11) **EP 2 250 985 A1**
(43) Date of publication of application: **17.11.2010**
(21) Application number: 10173073.7
(22) Date of filing: 29.04.2003
(51) Int. Cl.: A61F 13/15, A61F 13/74, A61F 13/76, A61F 13/72

(54) **Pull-on disposable wearing article**

(62) Divisional of application: 03252704.6
(71) Applicant: Uni-Charm Corporation, Ehime 799-0111 (JP)
(72) Inventor: Igaue, Takamitsu, Mitoyo-gun Kagawa 769-1602 (JP); Wada, Ichiro, Mitoyo-gun Kagawa 769-1602 (JP); Kurita, Noriyuki, Mitoyo-gun Kagawa 769-1602 (JP)
(74) Representative: Fitchett, Stuart Paul

(57) **Abstract**

A pull-on disposable wearing article 1A includes pants P1 having a waist-hole 2 and a pair of leg-holes 3, on one hand, and a liquid-absorbent pad P2 placed on the inner side of the pants P1. On the other hand, the pants P1 and the pad P2 are coupled together by means of first and second coupler sheets 21, 22 extending between the pants P1 and the pad P2. The pants P1 and first and second coupler sheets 21, 22 are elastically stretchable in a longitudinal direction as well as in a transverse direction. A stretch stress of the pants P1 is higher than those of the first and second coupler sheets 21, 22 and a stretch stress of the second coupler sheet 22 is lower than that of the first coupler sheet 21.

## Description

### BACKGROUND OF THE INVENTION

This invention relates to a pull-on disposable wearing article for absorption and containment of bodily discharges.

Japanese Patent Application No. 1997-75390A discloses a pull-on disposable wearing article basically having pants composed of front and rear waist regions opposed to each other and a crotch region extending between these waist regions so as to define a waist-hole and a pair of leg-holes, on one hand, and a liquid-absorbent pad placed on inside of the pants and extending over the crotch region into the front and rear waist regions. On the other hand, the pants have flexible first and second suspender sheets and the pad is detachably attached to these suspender sheets. In this article, the first and second suspender sheets are formed in appropriate zones thereof with a first fastener means and the pad is formed in front and rear end zones thereof with a second fastener means so that the second fastener means may be engaged with the first fastener means and thereby the pad may be attached to the first and second suspender sheets.

The pants are composed of substantially non-stretchable front and rear body sheets and a stretchable crotch sheet extending between these body sheets. Plural waist-surrounding elastic members extending in a transverse direction are contractibly attached to these front and rear body sheets, respectively, so that, during use of the article, these elastic members may retain the front and rear body sheets in close contact with the wearer's torso and thereby prevent the article from slipping down along the wearer's torso. The pad comprises a liquid-pervious topsheet facing the wearer's body, a liquid-impervious backsheet facing away from the wearer's body and a liquid-absorbent core interposed between these top- and backsheets.

In the wearing article disclosed in the above-cited Publication, the first and second suspender sheets are made of a nonwoven fabric or a plastic film or a spongy material having a cushioning property such as a polyurethane foam. This is, these suspender sheets substantially have no elastic stretchability and, during use of the article, it is impossible for these suspender sheets to retain the pad in close contact with the wearer's crotch. In addition, movement of the pants from the proper position thereof during use of the article is easily transmitted to the pad by means of the suspender sheets and consequently the pad moves together with the pants from the proper position thereof.

### SUMMARY OF THE INVENTION

It is an object of this invention to provide a pull-on disposable wearing article improved so that a movement of the article from a the proper position thereof during use of the article may be substantially prevented from being transmitted to a liquid-absorbent pad and thereby the pad may be retained in close contact with a wearer's crotch.

According to this invention, there is provided a pull-on disposable wearing article generally comprising pants composed of front and rear waist regions opposed to each other and a crotch region extending between the waist regions so as to define a waist-hole and a pair of leg-holes, on one hand, and a liquid-absorbent pad placed on inside of the pants and extending over the crotch region into the front and rear waist regions, on the other hand, the pad having a first coupler sheet extending outwardly from a front end portion of the pad in a longitudinal direction and a second coupler sheet extending outwardly from a rear end portion of the pad in the longitudinal direction, the first coupler sheet having an upper end portion attached to a waist-hole's peripheral edge portion in the front waist region and the second coupler sheet having an upper end portion attached to a waist-hole's peripheral edge portion in the rear waist region.

The article further comprises the pants and the first and second coupler sheets which are elastically stretchable in the longitudinal direction as well as in a transverse direction and the pants having a stretch stress higher than those of the first and second coupler sheets.

This invention includes the following embodiments. The stretch stress of the second coupler sheet is lower than that of the first coupler sheet.

The pants comprises elastically stretchable front and rear body sheets which are separately provided. The front and rear body sheets are joined at transversely opposite lateral portions and crotch end portions . The rear body sheet has a stretch stress lower than that of the front body sheet.

The pad comprises a liquid-pervious topsheet facing a wearer's body, a liquid-impervious backsheet facing away from the wearer's body and a liquid-absorbent core interposed between the top- and backsheets.

### BRIEF DESCRIPTION OF THE DRAWINGS

Fig. 1 is a partially cutaway perspective view showing a wearing article according to this invention;
Fig. 2 is a partially cutaway plan view showing the article with the front and rear waist regions of the pants having been disconnected from each other;
Fig. 3 is a sectional view taken along a line A - A in Fig. 1;
Fig. 4 is a sectional view taken along a line B - B in Fig. 1;
Fig. 5 is a partially cutaway perspective view showing another embodiment of the wearing article according to this invention;
Fig. 6 is a partially cutaway plan view showing the embodiment of the article with the front and rear waist regions of the pants having been disconnected from each other;
Fig. 7 is a sectional view taken along a line C - C in Fig. 5; and
Fig. 8 is a sectional view taken along a line D - D in Fig. 5.

### DETAILED DESCRIPTION OF THE PREFERRED EMBODIMENTS

Details of a pull-on disposable wearing article according to this invention will be more fully understood from the description given hereunder in reference to the accompanying drawings.

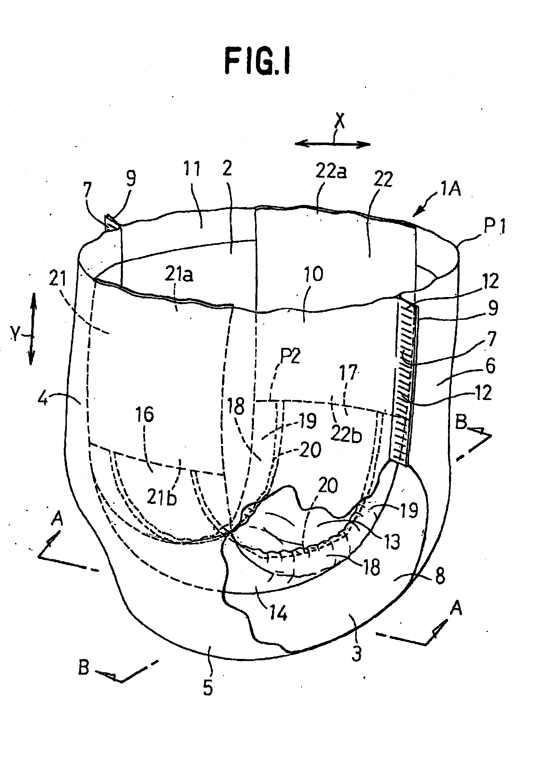

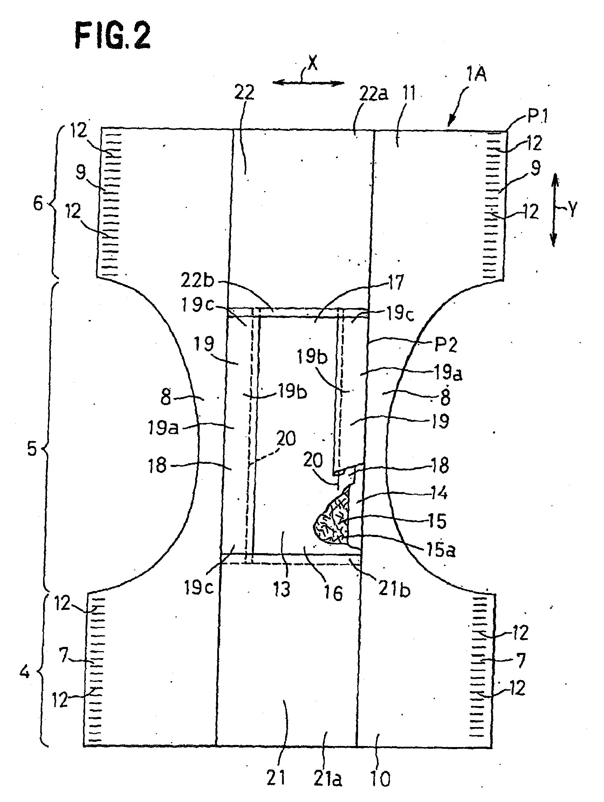

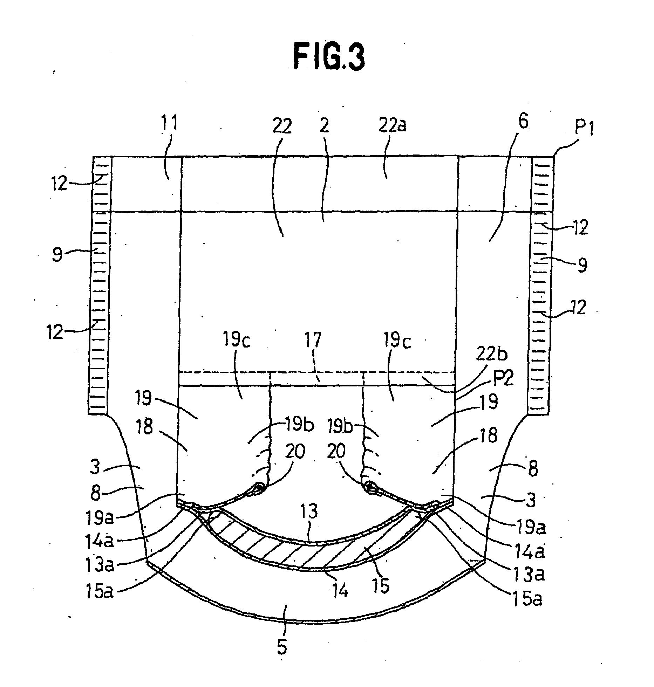

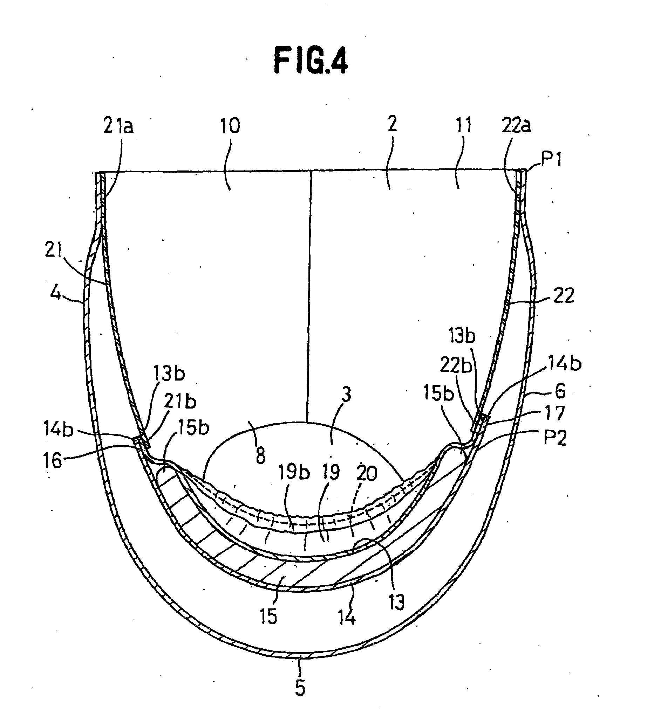

Fig. 1 is a partially cutaway perspective view showing a wearing article 1A according to this invention, Fig. 2 is a partially cutaway plan view showing this article 1A with the front and rear waist regions 4, 6 of the pants P1 having been disconnected from each other, Fig. 3 is a sectional view taken along a line A-A in Fig. 1 and Fig. 4 is a sectional view taken along a line B - B in Fig. 1. In Figs. 1 and 2, a transverse direction is indicated by an arrow X and a longitudinal direction is indicated by an arrow Y. Expression "inner surfaces of top-and backsheets 13, 14 and leak-barrier sheets 19" should be understood to designate the surfaces of these sheets facing a core 15 and expression "outer surfaces of these sheets 13, 14, 19" should be understood to designate the surfaces of these sheets facing away from the core 15.

The article 1A is composed of the pants P1 having a waist-hole 2 and a pair of leg-holes 3, a rectangular liquid-absorbent pad P2 placed on the inner side of the pants P1, and first and second coupler sheets 21, 22 serving to attach the pad P2 to the pants P1.

The pants P1 are composed of front and rear waist regions 4, 6 opposed to each other and a crotch region 5 extending between these waist regions 4, 6. In the pants P1, transversely opposite lateral portions 7 of the front waist region 4 and transversely opposite lateral portions 9 of the rear waist region 6 are overlaid and joined together by means of a plurality of heat-sealing lines 12 intermittently arranged in a longitudinal direction. In the crotch region 5 of the pants P1, leg-holes' peripheral edge portions 8 curve inwardly in a transverse direction of the pants P1. Thus the pants P1 present an hourglass-like planar shape. The pants P1 are made of a hydrophobic fibrous nonwoven fabric which is elastically stretchable in the longitudinal direction as well as in the transverse direction.

The pad P2 comprises a liquid-pervious topsheet 13 facing a wearer's body, a liquid-impervious backsheet 14 facing away from the wearer's body, and a liquid-absorbent core 15 interposed between these top- and backsheets 13, 14 and bonded to the inner surface of at least one of these sheets 13, 14. The pad P2 extends over the crotch region 5 into the front and rear waist regions 4, 6 of the pants P1 and is slightly spaced upwardly from the pants P1.

As seen in Fig. 2, the pad P2 has a front end portion 16 placed aside toward the front waist region 4 of the pants P1 and extending in the transverse direction, a rear end portion 17 placed aside toward the rear waist region 6 of the pants P1 and extending in the transverse direction, and transversely opposite lateral portions 18 extending in the longitudinal direction between these front and rear end portions 16, 17. A pair of liquid-impervious leak-barrier sheets 19 extending in the longitudinal direction are attached to the respective lateral portions 18 of the pad P2. The pad P2 is configured to have a transverse dimension between the opposite lateral portions 18 smaller than the minimum transverse dimension of the pants P1 in its crotch region 5.

The leak-barrier sheets 19 respectively have fixed lateral portions 19a extending in the longitudinal direction outside transversely opposite side edges 15a of the core 15, free lateral portions 19b extending in the longitudinal direction and normally biased to rise above the topsheet 13, and fixed longitudinally opposite end portions 19c lying on the front and rear end portions 16, 17 of the pad P2 and collapsed inwardly in the transverse direction. Stretchable elastic members 20 extending in the longitudinal direction are contractibly attached to the free lateral portions 19b of the respective leak-barrier sheets 19. These elastic members 20 are wrapped by parts of the respective free lateral portions 19b.

The first coupler sheet 21 is provided in the form of a vertically longer rectangular sheet and extends in the longitudinal direction between a waist-hole peripheral edge 10 in the front waist region 4 of the pants P1 and the front end portion 16 of the pad P2. The first coupler sheet 21 has its upper end portion 21a bonded to the waist-hole peripheral edge
10 of the front waist region 4 and its lower end portion 21b bonded to the front end portion 16 of the pad P2. The second coupler sheet 22 is provided also in the form of a vertically longer rectangular sheet and extends in the longitudinal direction between a waist-hole peripheral edge 11 in the rear waist region 6 of the pants P1 and the rear end portion 17 of the pad P2 . The second coupler sheet 22 has its upper end portion 22a bonded to the waist-hole peripheral edge 11 of the rear waist region 6 and its lower end portion 22b bonded to the rear end portion 17 of the pad P2. Both the first and second coupler sheets 21, 22 are made of a hydrophobic fibrous nonwoven fabric which is elastically stretchable in the longitudinal direction as well as in the transverse direction.

In the transversely opposite lateral portions 18 of the pad P2, the transversely opposite lateral portions 13a of the topsheet 13 extend outwardly slightly beyond the transversely opposite side edges 15a of the core 15 in the transverse direction. The transversely opposite lateral portions 14a of the backsheet 14 and the fixed lateral portions 19a of the leak-barrier sheets 19 extend outwardly beyond the lateral portions 13a of the topsheet 13 in the transverse direction. The lateral portions 13a of the topsheet 13 are interposed between the lateral portions 14a and the lateral portions 19a, respectively and bonded to the inner surfaces of these lateral portions 14a, 19a. The lateral portions 14a of the backsheet 14 and the lateral portions 19a of the leak-barrier sheets 19 are overlaid and joined together.

In the front and rear end portions 16, 17 of the pad P2, longitudinally opposite end portions 13b of the topsheet 13 and longitudinally opposite end portions 14b of the backsheet 14 extend outwardly beyond longitudinally opposite ends 15b of the core 15 in the longitudinal direction and overlaid and joined together. The fixed longitudinally opposite end portions 19c of the respective leak-barrier sheets 19 are bonded to the outer surface of the topsheet 13 at the longitudinally opposite end portions 13b thereof. The lower end portions 21b, 22b of the first and second coupler sheets 21, 22 are bonded to the outer surface of the topsheet 13 at the longitudinally opposite end portions 13b thereof and to the outer surface of the leak-barrier sheets 19 at the fixed longitudinally opposite end portions 19c thereof.

A stretch stress of the pants P1 is higher than those of the first and second coupler sheets 21, 22. A stretch stress of the second coupler sheet 22 is lower than that of the first coupler sheet 21. The first coupler sheet 21 is substantially identical to the second coupler sheet 22 with respect to longitudinal and transverse dimensions thereof.

The stretchable front and rear waist regions 4, 6 of the pants P1 are tightly placed over entire area thereof around the wearer's torso and the leg-holes' peripheral edge portions 8 tightly surround the wearer's legs as the article 1A is put on the wearer's body. Therefore, there is no inconvenience that the article 1A once put on the wearer's body might unintentionally slip down along the wearer's torso. In the article 1A, the stretchable first and second coupler sheets 21, 22 function to lift the pad P2 placed against the wearer's crotch from the crotch region 5 toward the waist-hole 2 so that the pad P2 may be kept in close contact with the wearer's crotch under a contractile force of these coupler sheets 21, 22 in the longitudinal direction.

In the article 1A put on the wearer's body, even if the pants P1 shift from a proper position, such a movement is effectively absorbed by the stretchable first and second coupler sheets 21, 22. Consequently, there is no possibility that the pad P2 might move together with the pants P1 and the pad P2 might shift from its initial position.

In the article 1A put on the wearer's body, even if the contractile force of the first and second coupler sheets 21, 22 in the longitudinal direction intends to pull the pants P1 downwardly along the wearer' s torso, the pants P1 can be retained in close contact with the wearer's torso and it is not likely that the pants P1 might slip down along the wearer's torso because the stretch stress of the pants P1 is higher than those of the coupler sheets 21, 22.

In the article 1A, the second coupler sheet 22 having the stretch stress lower than that of the first coupler sheet 21 stretches easily in the longitudinal direction in conformity with a particular contour of the wearer's hip and therefore there is no anxiety that the pad P2 might be pulled toward the wearer's hip under the contractile force of the second coupler sheet 22 in the longitudinal direction.

With the pad P2 curving in the longitudinal direction with the topsheet 13 inside, contraction of the elastic members 20 biases the free lateral portions 19b of the respective leak-barrier sheets 19 to rise above the topsheet 13. In the pad P2, the free lateral portions 19b of the respective leak-barrier sheets 19 for barriers against leakage of bodily discharges and thereby prevent bodily discharges from leaking sideways beyond the lateral portions 18 of the pad P2.

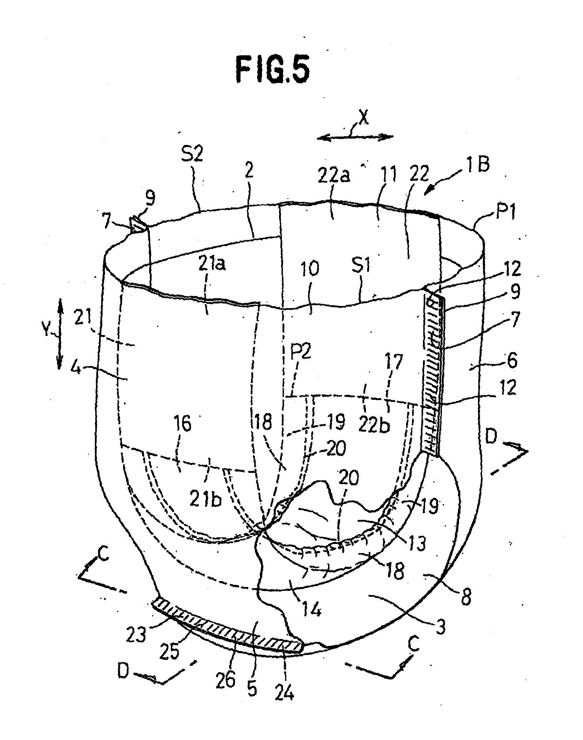

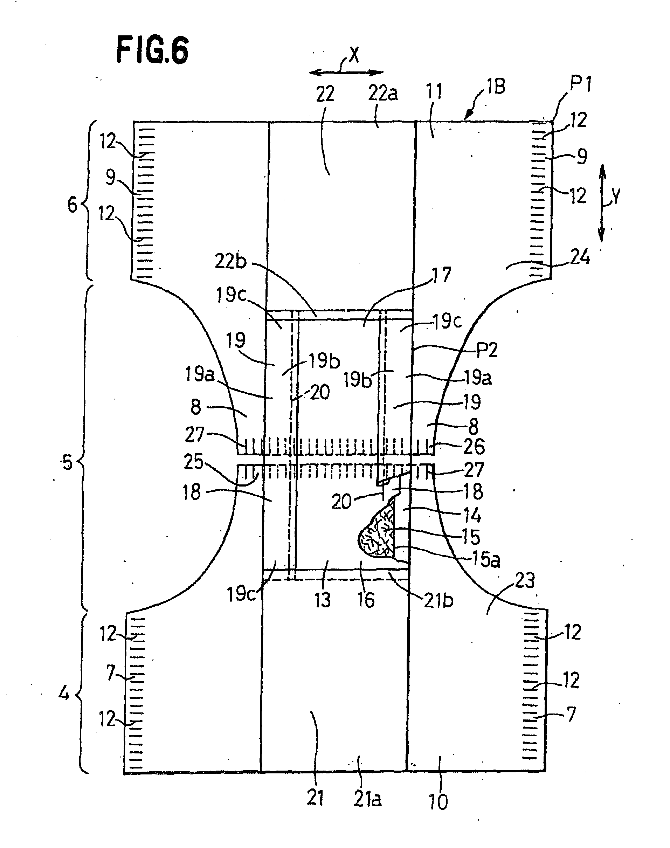

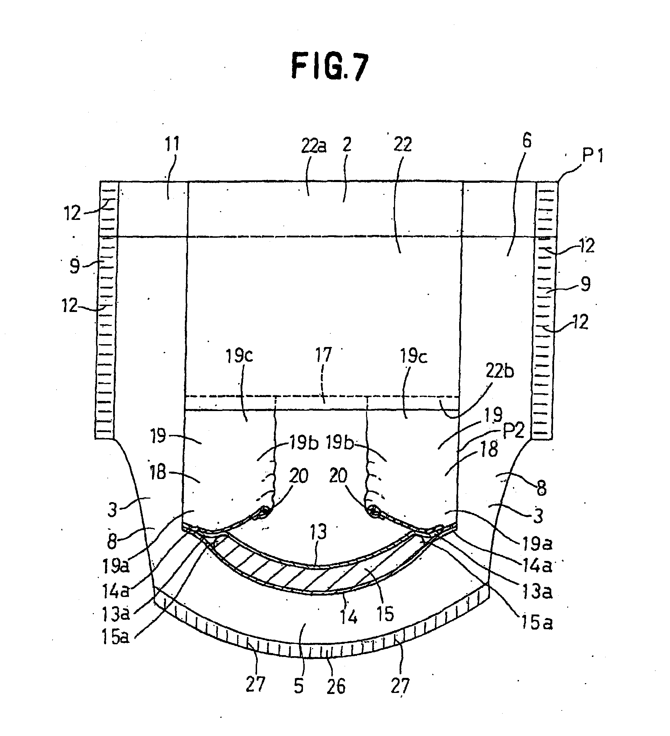

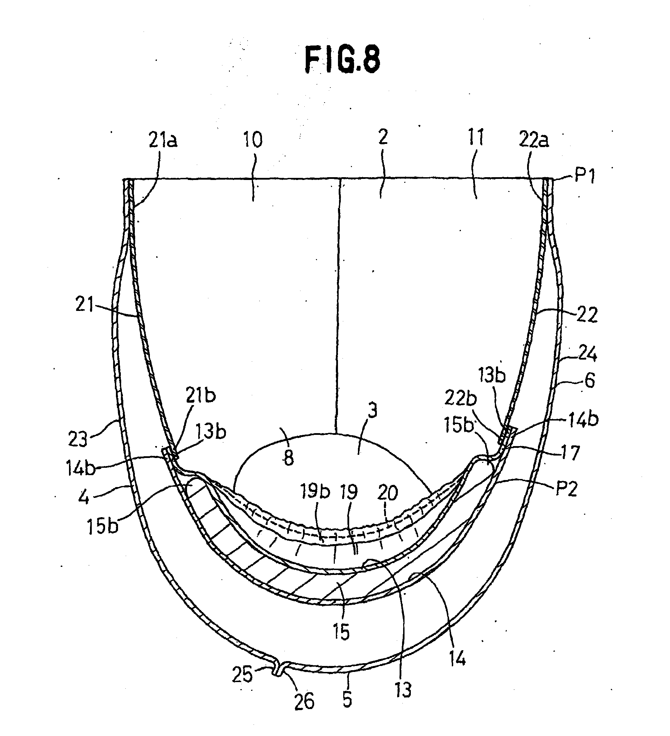

Fig. 5 is a partially cutaway perspective view showing another embodiment 1B of the wearing article according to this invention, Fig. 6 is a partially cutaway plan view showing the article 1B with the front and rear waist regions 4, 6 of the pants P1 having been disconnected from each other, Fig. 7 is a sectional view taken along a line C - C in Fig. 5 and Fig. 8 is a sectional view taken along a line D - D in Fig. 5. In Figs. 5 and 6 also, the transverse direction is indicated by the arrow X and the longitudinal direction is indicated by the arrow Y. In Fig. 6, crotch end portions 25, 26 of front and rear body sheets 23, 24 are illustrated as have been disconnected from each other.

The article 1B is composed of the pants P1 having a waist-hole 2 and a pair of leg-holes 3, a liquid-absorbent pad P2 placed on the inner side of the pants P1, and first and second coupler sheets 21, 22 serving to attach the pad P2 to the pants P1.

The pants P1 comprises a front body sheet 23 and a rear body sheet 24 which are separately provided and elastically stretchable and have front and rear waist regions 4, 6 opposed to each other and a crotch region 5 extending between these waist regions 4, 6.

In the pants P1, transversely opposite lateral portions 7 of the front body sheet 23 and transversely opposite lateral portions 9 of the rear body sheet 24 are overlaid and joined together by means of a plurality of heat-sealing lines 12 intermittently arranged in the longitudinal direction. In the pants P1, the crotch end portion 25 of the front body sheet 23 and the crotch endportion 26 of the rear body sheet 24 are overlaid and joined together by means of a plurality of welding lines 27 arranged intermittently in the transverse direction. The front body sheet 23 and the rear body sheet 24 are made of a hydrophobic fibrous nonwoven fabric which is elastically stretchable in the longitudinal direction as well as in the transverse direction. In the pants P1, a stretch stress of the rear body sheet 24 is lower than that of the front body sheet 23.

The pad P2 comprises a liquid-pervious topsheet 13, a liquid-impervious backsheet 14, and a liquid-absorbent core 15 interposed between these top- and backsheets 13, 14. The pad P2 extends over the crotch region 5 into the front and rear waist regions 4, 6 of the pants P1 and is slightly spaced upwardly from the pants P1. As seen in Fig. 6, the pad P2 has front and rear end portions 16, 17 and transversely opposite lateral portions 18. A pair of liquid-impervious leak-barrier sheets 19 extending in the longitudinal direction are attached to the respective lateral portions 18 of the pad P2. The pad P2 is configured to have a transverse dimension between the opposite lateral portions 18 smaller than the minimum transverse dimension of the pants P1 in its crotch region 5.

The first coupler sheet 21 is provided in the form of a vertically longer rectangular sheet and bonded at its upper end portion 21a to the waist-hole peripheral edge portion 10 in the front waist region 4 and bonded at its lower end 21b to the front end portion 16 of the pad P2. The second coupler sheet 22 is provided also in the form of a vertically longer rectangular sheet and bonded at its upper end portion 22a to the waist-hole peripheral edge portion 11 in the rear waist region 6 and bonded at its lower end portion 22b to the rear end portion 17 of the pad P2. Both the first and second coupler sheets 21, 22 are made of a hydrophobic fibrous nonwoven fabric which is elastically stretchable in the longitudinal direction as well as in the transverse direction.

A stretch stress of the pants P1 is higher than those of the first and second coupler sheets 21, 22. A stretch stress of the second coupler sheet 22 is lower than that of the first coupler sheet 21. The first coupler sheet 21 is substantially identical to the second coupler sheet 22 with respect to longitudinal and transverse dimensions thereof.

The stretchable front and rear waist regions 4, 6 of the pants P1 are tightly placed over entire area thereof around the wearer's torso and the leg-holes' peripheral edge portions 8 tightly surround the wearer's legs as the article 1B is put on the wearer's body. Therefore, there is no inconvenience that the article 1B once put on the wearer's body might unintentionally slip down along the wearer's torso.

In the article 1B, the stretchable first and second coupler sheets 21, 22 function to lift the pad P2 placed against the wearer's crotch from the crotch region 5 toward the waist-hole 2 so that the pad P2 may be kept in close contact with the wearer's crotch. In the article 1B put on the wearer's body, even if the pants P1 shift from a proper position, such movement is effectively absorbed by the stretchable first and second coupler sheets 21, 22. Consequently, there is no possibility that the pad P2 might move together with the pants P1 and the pad P2 might shift from its initial position.

In the article 1B put on the wearer's body, even if the contractile force of the first and second coupler sheets 21, 22 in the longitudinal direction intends to pull the pants P1 downwardly along the wearer's torso, it is not likely that the pants P1 might slip down along the wearer's torso because the stretch stress of the first and second coupler sheets 21, 22 is lower than that of the pants P1 . In the article 1B, the second coupler sheet 22 having the stretch stress lower than that of the first coupler sheet 21 stretches easily in the longitudinal direction in conformity with a particular contour of the wearer's hip and therefore there is no anxiety that the pad P2 might be pulled toward the wearer's hip under the contractile force of the second coupler sheet 22.

In the article 1B, the rear body sheet 24 having a stretch stress lower than that of the front body sheet 23 stretches easily in the transverse direction as well as in the longitudinal direction so that the pants P1 can be made in conformity with the particular body contour of the wearer's body. In the article 1B, the free lateral portions 19b of the respective leak-barrier sheets 19 for barriers against leakage of bodily discharges and thereby prevent bodily discharges from leaking sideways beyond the lateral portions 18 of the pad P2.

A stock material for the topsheet 13 may be selected from the group consisting of a hydrophilic fibrous nonwoven fabric, a hydrophobic fibrous nonwoven fabric having a plurality of perforations and a plastic film having a plurality of fine perforations.

A stock material for the backsheet 14 and the leak-barrier sheets 19 may be selected from the group consisting of a substantially non-stretchable hydrophobic fibrous nonwoven fabric, a substantially non-stretchable breathable but liquid-impervious plastic film, a composite nonwoven fabric comprising two or more hydrophobic fibrous nonwoven fabric layers laminated one with another and a composite sheet of a hydrophobic fibrous nonwoven fabric and a breathable but liquid-impervious plastic film laminated with each other.

It is also possible to use, as a stock material for the backsheet 14 and the leak-barrier sheets 19, a composite nonwoven fabric comprising a melt blown fibrous nonwoven fabric having a high water-resistance and two layers of spun bond fibrous nonwoven fabrics, each having a high strength and a high flexibility, sandwiching the melt blown fibrous nonwoven fabric therebetween.

The type of nonwoven fabric to be used may be selected from the group consisting of spun lace-, needle punch-, melt blown- thermal bond-, spun bond-, chemical bond- and air-through-types. Component fibers of the nonwoven fabric may be selected from the group consisting of polyolefine-, polyester- and polyamide-based fibers, and a core-sheath type or a side-by-side type conjugated fiber of polyethylene/polypropylene or polyethylene/polyester.

The stretchable nonwoven fabric as a stock material for the pants P1 and the first and second coupler sheets 21, 22 may be either a melt blown nonwoven fabric or a spun bond nonwoven fabric. Component fibers of such a stretchable nonwoven fabric may be stretchable fibers obtained by melt-spinning a suitable thermoplastic elastomer resin.

A stock material for the pants P1 and the first and second coupler sheets 21, 22 is not limited to the stretchable hydrophobic fibrous nonwoven fabric but may be selected also from the group consisting of an elastically stretchable liquid-impervious plastic film, a composite nonwoven fabric comprising two stretchable hydrophobic fibrous nonwoven fabric layers laminated with each other and a composite sheet comprising a stretchable hydrophobic fibrous nonwoven fabric and a stretchable liquid-impervious plastic film laminated with each other.

As a stock material for the pants P1 and the first and second coupler sheets 21, 22, it is possible to use a composite nonwoven fabric comprising a stretchable hydrophobic fibrous nonwoven fabric made of thermoplastic elastomer resin fibers and a hydrophobic fibrous nonwoven fabric made of crimped fibers obtained by melt-spinning a suitable thermoplastic synthetic resin such as polypropylene, polyethylene or polyester wherein the latter is laminated on at least one surface of the former.

The core 15 is a mixture of fluff pulp and super-absorbent polymer particles or a mixture of fluff pulp, super-absorbent polymer particles and thermoplastic synthetic resin fibers, in both cases, compressed to a desired thickness. Preferably the core 15 is entirely covered with a liquid-pervious sheet such as a tissue paper or a hydrophilic fibrous nonwoven fabric in order to prevent the core 15 from getting out of its initial shape or to avoid falling off of the polymer particles from the core 15. The polymer particles may be particles of starch-, cellulose- or synthetic polymer.

Joining of the sheets 13, 14, 19, 23, 24 one to another, attachment of the core 15 to the sheets 13, 14, bonding of the first and second coupler sheets 21, 22 to the pants P1 and the pad P2, and attachment of the elastic members 20 to the sheet 19 may be carried out using hot melt adhesive agents or welding techniques such as heat-sealing or sonic sealing.

In the pull-on disposable wearing article according to this invention, the stretchable first and second coupler sheets cooperate with each other to lift the pad placed against the wearer's crotch toward the waist-hole so that the pad can be retained in close contact with the wearer's crotch under the contractile force of these coupler sheets in the longitudinal direction. In this article, even if the pants shift from the initial position at which the pants have been put on the wearer's body, such movement can be satisfactorily absorbed by the first and second coupler sheets. Thus, it is avoided that the pad might move together with the pants and the pad might shift from its initial position. During use of this article, even if a contractile force of the first and second coupler sheets in the longitudinal direction intend to pull the pants downwardly along the wearer's torso, the pants can be retained in close contact with the wearer's torso and there is no anxiety that the pants might slip down along the wearer's torso because the stretch stress of the pants is higher than the stretch stress of these coupler sheets.

In the case of the article wherein the stretch stress of the second coupler sheet is lower than the stretch stress of the first coupler sheet, the second coupler sheet stretches easily in conformity with the particular contour of the wearer. Consequently, it is reliably avoided that the pad might be drawn toward the wearer's hip.

In the case of the article wherein the pants comprises the elastically stretchable front and rear body sheets which are separately provided, the rear body sheet having a stretch stress lower than that of the front body sheet stretches easily in the longitudinal direction as well as in the transverse direction in conformity with the particular contour of the wearer' hip so that the pants can be made in conformity with the wearer's particular body contour.

## Claims

1. A pull-on disposable wearing article (1A, 1B) having a longitudinal direction (Y) and a transverse direction (X) comprising:
pants (P1) comprising front and rear waist regions (4, 6) opposed to each other and a crotch region (5) extending between said front and rear waist regions (4, 6) so as to define a waist hole (2) and a pair of leg-holes (3),
a liquid-absorbent pad (P2) placed on the inside of said pants (P1) and extending over at least said crotch region (5) into said front and rear waist regions, said pad (P2) having a first coupler sheet (21) extending outwardly from a front end portion (16) of said pad (P2) in said longitudinal direction (Y) and a second coupler sheet (22) extending outwardly from a rear end portion (17) of said pad (P2) in said longitudinal direction (Y), said first coupler sheet (21) having an upper end portion (21a) attached to a waist-hole's peripheral edge portion (10) in said front waist region, said second coupler sheet (22) having an upper end portion (22a) attached to waist-hole's peripheral edge portion (11) in said rear waist region (6); said wearing article (1A, 1B) being **characterized in that**:
said pants (P1) and said first and second coupler sheets (21, 22) are elastically stretchable in said longitudinal direction (Y) as well as in a transverse direction (X).

2. The wearing article (1A, 1B) according to Claim 1, wherein said pants (P1) have a stress stretch higher than those of said first and second coupler sheets.

3. The wearing article (1A, 1B) according to Claim 1 or 2, wherein the stretchable first and second coupler sheets (21, 22) function to lift the pad (P2) placed against the wearer's crotch from the crotch region (5) toward the waist-hole (2) so that the pad (P2) may be kept in close contact with the wearer's crotch under a contractile force of these coupler sheets (21, 22) in the longitudinal direction.

4. The wearing article (1A, 1B) according to Claim 1, 2 or 3, wherein said pad (P2) has a transverse dimension smaller than a minimum transverse direction of said crotch region (5) and a longitudinal dimension substantially equal to that of said crotch region (5).

5. The wearing article (1A, 1B) according to any one of Claims
1 to 4, wherein said first coupler sheet (21) is shaped into a vertically longer rectangle having transverse dimension substantially equal to the transverse dimension of said pad (P2) and has said upper end portion (21a) attached to a transverse middle zone of said waist-hole's peripheral edge portion (10) in said front waist region (4) and a lower end portion (21b) bonded to said front end portion (16) of said (P2); and
said second coupler sheet (22) is shaped into a vertically longer rectangle having a transverse dimension substantially equal to the transverse dimension of said pad (P2) and has said end (22a) attached to a transverse middle zone of said waist-hole's peripheral edge portion (11) in said rear waist region (6) and a lower end portion (22b) bonded to said rear end portion (17) of said pad (P2).

6. The wearing article (1B) according to any one of Claims 1 to 5, wherein saidpad (P2) comprises a liquid-pervious topsheet (13) facing a wearer's body, a liquid-impervious backsheet (14) facing away from said wearer's body and a liquid-absorbent core (15) interposed between said top- and backsheets (13, 14).

7. The wearing article (1A, 1B) according to any one of Claims 1 to 6, wherein said pad (P2) further comprises a pair of liquid pervious leak-barrier sheets (19) each having a fixed lateral portion (19a), an elasticized free lateral portion (19b) and a longitudinal end portion (19c), wherein said fixed lateral portion (19a) is fixed along a corresponding lateral portion of said pad (P2) and said free lateral portion (19b) is biased to rise above said topsheet (13) and lower end portions (21b, 22b) of said first and second coupler sheets (21, 22) are bonded to the outer surface of said leak-barrier sheets (19), at said longitudinal opposite end portions (19c).
